# EUROPEAN PATENT APPLICATION

(11) **EP 4 512 472 A2**
(43) Date of publication of application: **26.02.2025**
(21) Application number: 24212151.5
(22) Date of filing: 31.07.2020
(51) Int. Cl.: A61P 25/00

(54) **TOPICAL FORMULATIONS COMPRISING CANNABIDIOL, METHOD OF PREPARING THE COMPOSITION AND USE THEREOF**

(30) Priority: 05.08.2019 DK PA201970497; 28.05.2020 DK PA202070343
(62) Divisional of application: 20757830.3
(71) Applicant: CS Medica A/S, 2100 Copenhagen (DK)
(72) Inventor: HENRIKSEN, Lone, 3480 Fredensborg (DK); DUGUINE, Maria Agustina, 08021 Barcelona (ES); DAUER, Rosemarie, 91352 Hallerndorf (DE)
(74) Representative: Bak, Poul Erik

(57) **Abstract**

A hydroalcoholic gel composition formulated for topical use is disclosed for alleviating symptoms of psoriasis or arthritis, such as pain resulting from arthritic diseases and/or psoriatric arthritis and/or neurological pain, such as pain resulting from sclerosis, e.g. multiple sclerosis in mammals. Such composition may comprise, *inter alia,* 0.1-20 % cannabidiol, a skin penetration enhancer, ethanol, and present in an amount of 0.4-2 % (by weight), one or more thickeners or gelling agents, and water in a quantity for the composition to a total of 100 % (by weight).

## Description

### Field of the Invention

The present invention relates to topical hydroalcoholic gel compositions comprising cannabidiol for use as cosmetic agent and/or to produce a medical composition in local topical application in the treatment or alleviation of symptoms of psoriasis or symptoms, such as pain, resulting from arthritic diseases and/or psoriatric arthritis and/or neurological pain, such as pain resulting from sclerosis, e.g. multiple sclerosis, in mammals.

### Background of the Invention

The clinical usefulness of various cannabinoids is well-known to provide analgesia and neuroprotection, help alleviate nausea and emesis, as well as treat epilepsy, anxiety disorders, and glaucoma.

Cannabidiol ("CBD") is also well-recognized, in particular for its mild analgesic effect as well as its anti-inflammatory effects. In addition, it is also well-known that cannabidiol lacks the psychoactive effects seen in many of the other cannabinoids, including Δ<9>-tetrahydrocannabinol (THC). The latter (THC) is currently available in an oral dosage form, sold under the trade name Marinol^{®}.

Many diseases relates to the inflammatory processes that are regulated in the individual by the immune system. Inflammation may result from an overactive immunoresponse, such as an allergic reaction or dermatitis, auto-immunoresponse, such as certain forms of multiple sclerosis, inflammatory bowel disorders and arthritis. Regardless of the underlying cause of the inflammation, it is therapeutically desirable under these circumstances to regulate the immune system and lessen the inflammatory response leading to these diseases.

Psoriasis is a non-contagious, long-lasting autoimmune disease characterized by patches of abnormal skin, typically by small, localized patches or larger areas (e.g. on the entire body) of red, dry, itchy, and/or scaly skin. Injuries to the skin may trigger psoriatic skin changes (Koebner phenomenon).

The underlying mechanism is believed to involve the immune system reacting to skin cells which results in an excessive hyperproliferation of keratinocytes. Keratinocytes constitutes approx. 90% of the epidermis. In normal skin, cell proliferation of the epidermis cells result in a regeneration cycle of approx. 30 days whereas in psoriasis affected skin areas, the cell proliferation has a regeneration cycle of as little as 3-5 days.

There are five main types of psoriasis: plaque, guttate, inverse, pustular, and erythrodermic. Plaque psoriasis typically presents as red patches with white scales on top. The affected area is typically the back of the forearms, shins, navel area, and scalp or face. Fingernails and toenails are often also affected by psoriasis at some point in time and may cause pits in the nails and/or changes in nail color. Pustular psoriasis is seen as small (non-infectious) pus-filled blisters. Inverse psoriasis forms red patches in skin folds. Erythrodermic psoriasis can develop from any of the other types and typically occurs when the rash becomes very widespread.

Psoriasis is generally thought to be a genetic disease that is triggered by environmental factors. It is suggested that genetic factors predispose to psoriasis. Symptoms may worsen during winter and with intake/use of certain medications, such as beta blockers or NSAIDs. Infections and psychological stress can also play a role.

There is currently no cure for psoriasis; however, various treatments can help control the symptoms. Treatments include steroid creams, vitamin D3 cream, ultraviolet light and immune system suppressing medications (e.g. methotrexate). Often symptoms of psoriasis can be managed with creams alone. Psoriasis is associated with an increased risk of psoriatic arthritis, lymphomas, cardiovascular disease, Crohn's disease and depression. Psoriatic arthritis affects up to 30 percent of individuals with psoriasis

Rheumatoid arthritis, psoriatic arthritis and osteoarthritis cause pain in the joints affected and the pain associated with rheumatoid arthritis can be disabling.

Certain cannabinoids have been shown to regulate various steps in the immune response and could show some therapeutic benefit in the treatment of certain inflammatory diseases.

Cannabinoids have been found to be useful as an adjunct treatment for rheumatoid arthritis, psoriatic arthritis and osteoarthritis and joint pain secondary to other autoimmune diseases, such as inflammatory bowel disease, multiple sclerosis and systemic lupus erythematosus.

The cannabinoids are usually administered orally and thus affect the individual systemic. In addition to the above-discussed therapeutics benefits, cannabionids present a variety of pharmacological benefits, including, but not limited to, anti-inflammatory, anti-convulsant, anti-psychotic, antioxidant, neuroprotective, anti-cancer and immunomodulatory effects.

When considering these systemic therapeutic benefits, it would be advantageous to develop a composition in which cannabidiol (CAS no: 3956-29-1) is delivered systemically to achieve therapeutically effective plasma concentrations in a patient. However, cannabinoid oral dosage forms, including cannabidiol, must overcome several obstacles in order to achieve a systemic concentration. First, cannabinoids, including cannabidiol, are generally highly lipophilic. Their limited water solubility thereby restricts the amount of cannabinoid available for absorption in the gastrointestinal tract.

Cannabidiol, as with the other cannabinoids, undergoes metabolism when absorbed from the human gastrointestinal tract. Thus, the overall effective uptake of orally administered cannabinoids to an individual, such as cannabidiol, is varying from individual to individual and it is difficult to control dosage thereof. Thus in reality, by oral administration of cannabinoids it is very difficult to achieve therapeutically effective plasma concentrations in a patient, and this may lead to some individuals being treated with a dose that is too high while other individuals are administered a dose that is lower than necessary to achieve therapeutically effective plasma concentrations.

Thus, it would be desirable to provide a delivery method to deliver therapeutically effective amounts of cannabidiol to a mammal in need thereof for the local treatment of one or more medical conditions responsive to cannabidiol, in particular skin conditions related to psoriasis or pain related to arthritis, as mentioned above, by providing a route of administration that does not depend upon absorption from the gastrointestinal tract of the mammal and is not subject to first-pass metabolism upon absorption from the gastrointestinal tract. One non-oral route of administration for the systemic delivery of cannabidiol is transdermal administration.

Unfortunately, due to its highly hydrophobic nature, cannabidiol is poorly absorbed through membranes such as the skin of mammals, including humans. Therefore, the success of transdermal administering therapeutically effective quantities of cannabidiol to a mammal in need of such treatment within a reasonable time frame has been rather limited.

In order to increase skin penetration it has been proposed to administer prodrugs of cannabidiol and/or other cannabinoids. The prodrugs are then partially metabolized in the epidermis upon penetration into the epidermis, and are then converted into e.g. cannabidiol and a byproduct after penetration into the epidermis. These byproducts are, however, unwanted as their physiological effects are unknown and may cause unknown or unwanted side effects.

Therefore, there is still a need for significant advancement in the development of a composition suitable for topical compositions for cosmetic conditioning of the skin.

There is in particular still a need for significant advancement in the development of a composition suitable for topical delivery of cannabidiol to improve availability of cannabidiol at the local administration site of a mammal in a therapeutically effective amount necessary to reduce or alleviate local symptoms of psoriasis or arthritis, such as rheumatiod arthritis, juvenile rheumatoid arthritis, osteoarthritis and/or psoriatric arthritis.

Surprisingly and unexpectedly, the inventors have been able to provide compositions that are solving current needs and limitations of the prior art.

### Summary of the Invention

The object of the present invention can thus be seen as the provision of novel compositions, such as topical compositions, in particular:
(I) Cosmetic compositions to improve the overall cosmetic appearance or condition, such as moisturizing /hydration, reducing tendency to dry skin, redness, "winter prone" skin, and/or reduce formation of wrinkles and/or reducing further development of wrinkles of otherwise healthy skin in subject, such as a human, mammal or other animal;
(II) Medical compositions for topical delivery of active ingredients to improve availability of the active ingredients, including cannabidiol, menthol, camphor and/or eucalyptus oil, at the local administration site of a subject, such as a human, mammal or other animal, in a therapeutically effective amount necessary to reduce or alleviate local symptoms of psoriasis and/or arthritis, such as rheumatoid arthritis, juvenile rheumatoid arthritis, osteoarthritis, or psoriatric arthritis and/or neurological pain, such as pain resulting from Sclerosis, in particular multiple sclerosis, and
(III) Medical compositions suitable for topical delivery of active ingredients, including cannabidiol, menthol, camphor and/or eucalyptus oil, to improve availability of the active ingredients at the local administration site of a subject, such as a human, mammal or other animal in a therapeutically effective amount necessary to reduce or alleviate local symptoms such as inflammation of skin or joints, local pain, red, dry and/or irritated skin, pruritus.

In a first aspect, the present invention relates to a hydroalcoholic gel composition comprising
a. cannabidiol present in an amount of 0.1-20%, such as 0.1-10% or more preferred 0.2-5 % (by weight),
b. a skin penetration enhancer present in an amount of 0.5-1.5% (by weight),
c. ethanol present in an amount of 10-30 % (by weight)
d. one or more thickeners or gelling agents present in a total amount of 0.4-2 % (by weight), and
e. water in a quantity for the composition to a total of 100% (by weight).

In some embodiments, the cannabidiol is provided in a crystalline or pure form, such as a cannabidiol preparation comprises less than 1.5%, 1.0% or 0.5% (by weight) of any one of: Cannabidivarin (CBDV), Cannabidiolic acid (CBDA), Cannabigerol (CBG), Cannabinol (CBN); and/or less than 1.0% of Tetrahydrocannabinol (THC).

In a second aspect, the present invention pertains to a composition according to the first aspect for use as a medical composition in a local topical application in the treatment or alleviation of symptoms, such as pain, resulting from arthritis, in particular rheumatoid arthritis, osteoarthritis, juvenile rheumatoid arthritis and/or psoriatric arthritis and/or neurological pain, such as pain resulting from sclerosis, e.g. multiple sclerosis, in a subject, such as human, mammal or other animal.

In a third aspect, the present invention concerns to the use of a composition according to the first or second aspect as a medicament, such as a medicament for treatment or alleviation of symptoms, such as pain, resulting from arthritis, in particular rheumatoid arthritis, osteoarthritis, juvenile rheumatoid arthritis and/or psoriatric arthritis and/or neurological pain, such as pain resulting from sclerosis, e.g. multiple sclerosis, in a subject, such as human, mammal or other animal.

In a fourth aspect, the present invention relates to a kit comprising a composition according to the first or second aspect, including instruction for use.

### Detailed description of the Invention

### Definitions:

In the context of the present invention, the singular form of a word may include the plural, and vice versa, unless the context clearly dictates otherwise. Thus, the references "a," "an" and "the" are generally inclusive of the plurals of the respective terms. For example, reference to "an ingredient" or "a method" may include a plurality of such "ingredients" or "methods."

Similarly, the words "comprise," "comprises," and "comprising" are to be interpreted inclusively rather than exclusively. Embodiments provided by the present disclosure may lack any element that is not specifically disclosed herein. Thus, a disclosure of an embodiment defined using the term "comprising" is also a disclosure of embodiments "consisting essentially of" and "consisting of the disclosed components". Where used herein, terms like "for example", "e.g." or "such as", particularly when followed by a listing of terms, is merely exemplary and illustrative, and should not be deemed to be exclusive or comprehensive. Any embodiment disclosed herein may be combined with any other embodiment disclosed herein.

The phrase "in some embodiments" can be used interchangeably with "in one or more embodiments".

Unless expressed otherwise, all percentages expressed herein are by weight of the total weight of the composition.

As used herein, "about" is understood to refer to numbers in a range of numerals, for example the range of +/- 10, +/- 5, +/- 2, +/- 1, +/- 0.5, +/- 0.1 % of the referenced number. In some embodiments, the range is +/- 20 % of the referenced number. Moreover, all numerical ranges herein should be understood to include all integers, whole or fractions, within the range. "About" may also indicate the variations and/or uncertainties customary in the field.

A "subject" can e.g. be a human or an animal, such as a mammal, bird, reptile, husbandry, or pet. The present disclosure should not be construed as being limited to a specific animal, human and/or demography. In some embodiments, the subject is a human. In some embodiments, the subject is an animal. In some embodiments, the animal is a mammal, such as a cat, dog, or horse. In some embodiments, the animal is a bird, reptile, husbandry or pet. In some embodiments, the subject is a human, such as an infant, child, adolescent, adult, or senior.

The present composition has a positive effect on the normalisation affected skin in psoriasis prone skin areas. Thus, local application of the composition according to the present invention for a period reduces or even eliminates symptoms of psoriasis (red spots, itch/pruritus and scaly skin) in affected in areas. Initial tests indicate that topical use for a period of 1-3 weeks of daily use with 1-5 daily applications of the hydroalcoholic gel on the affected skin area reduces skin redness, scaly appearance of the spots and greatly reduces itch/pruritus at the affected skin areas. The hydroalcoholic gel causes proliferation activity inhibition. Since psoriasis is an inflammatory skin disease which produces an excessive hyperproliferation of keratinocytes, the hydroalcoholic gel according to the present invention could be beneficial for the treatment of this disease.

The present composition further appears to have a positive effect in local treatment of rheumatism. Thus, local application of the composition according to the present invention reduces pain from joints affected with rheumatism. Thus, the present composition may be used as a locally applied mild analgesic and may thus reduce the patient's intake of other NSAIDS or of other prescribed drugs against rheumatism. In mild cases of rheumatism or in periods with low pain levels in patients, the present composition may even replace NSAIDs or other drugs taken against the patient's rheumatism. Similarly, present composition further appears to have a positive effect in local treatment of neurological pain, such as pain resulting from sclerosis, e.g. multiple sclerosis.

The present composition has a positive effect in treatment, alleviation and/or reduction of a condition in a subject, such as a mammal, and in particular in a human being. Said condition may comprise psoriasis and/or psoriasis-related conditions, such as or arthritis, rheumatism, and/or arthritis and/or rheumatism-related conditions, such as joint pain, swollen joints and the like. Main effects of CBD on arthritis- and/or psoriasis-related symptoms are given in Table A:

**Table A Main effects of CBD and symptoms of Arthritis and Psoriasis**

| **CBD effect** | **Arthritis symptoms** | **Psoriasis symptoms** |
|---|---|---|
| **Analgesic(reduce pain)** | Pain | Pain |
| **Anti inflammation** | Inflammation | Inflammation (redness skin) |
| **Bone stimulant** | Bone degradation | |
| **Immunosuppressive** | Immune system attack - RA | Immune system attack the skin |
| **Antiproliferative** | | faster skin cells reproduction |
| **Antioxydant** | | oxidative stress |
| **Reduce itching** | | Itch |
| **homeostatic regulator** | Immune system attack - RA | Immune system attack the skin |

The hydroalcoholic gel is further non-toxic and increases the transdermal delivery of cannabidiol in local application to the skin. Absorption of cannabidiol through the skin/epidermis may be as high as above 46% of the applied cannabidiol. In comparison, when administering cannabidiol orally, less than 10% is absorbed through the gastrointestinal passage.

Thereby, the cannabidiol becomes more readily available at the local area in the epidermis and/or in tissue underneath the skin, e.g. tendons, muscles, cartilage etc., where the hydroalcoholic gel is applied. This reduces or eliminates any side effects caused by oral administration and reduces costs, as the amount of cannabidiol that is administered topically and locally to a person is greatly reduced (relative to oral administration) without reducing the local effect of cannabidiol in relation to alleviation of symptoms from psoriasis and/or different types of arthritis.

The hydroalcoholic gel is also excellent for dissolving cannabidiol as cannabidiol is lipophilic and thus readily dissolved in ethanol, while cannabidiol is poorly dissolvable in aqueous media. Thus, the dissolution of cannabidiol can be made by first dissolving the cannabidiol in ethanol and then mix the cannabidiol-containing ethanol fraction with an aqueous fraction that comprises the remaining ingredients to prepare the hydroalcoholic gel. Further, since ethanol is a good solvent for cannabidiol, the presence of ethanol in the hydroalcoholic gel also acts as skin penetration enhancer for cannabidiol.

Cannabidiol is present in an amount of 0.1-20%, such as 0.1-10%. In vitro experiments show that a concentration of 0.1 - 5 % (by weight), in particular 1% or more of cannabidiol in the hydroalcoholic gel provides the therapeutic effects described above.

The hydroalcoholic gel further comprises at least one skin penetration enhancer present in an amount of 0.5-1.5% (by weight) to further enhance skin penetration of the highly lipophilic cannabidiol and/or other active ingredients, including menthol, camphor and/or eucalyptus oil. Therefore, the preferred penetration enhancer is an oily substance. The preferred penetration enhancers comprise e.g.: isopropyl myristate (Cas reg. no: 110-27-0), g. dimethylsulfoxid (DMSO) ( Cas re. no:67-68-5), urea/ car-bamide (Cas reg. no: 57-13-6) or any combinations thereof. The most preferred penetration enhancer is isopropyl myristate. In some embodiments, the hydroalcoholic gel comprises 0.5-1.5% (by weight) of skin penetration enhancer selected from: isopropyl myristate, dimethylsulfoxid (DMSO), urea, and any combinations thereof.

The specific combination of isopropyl myristate and ethanol present in the hydroalcoholic gel greatly enhances skin penetration of cannabidiol. Ethanol is also enhancing skin penetration of cannabidiol. It is believed that skin contact with ethanol creates micro-cracks in the skin. The formation of micro-cracks allows the other penetration enhancers, such as isopropyl myristate, and/or camphor, menthol etc. as described further below to exhibit increased skin penetration. Thereby skin penetration of cannabidiol is also further increased.

Ethanol present in an amount of 10-30 % (by weight) in the hydroalcoholic gel. Preferably, the hydroalcoholic gel comprises 15-25% (by weight) of ethanol.

In order to provide a gel or gel-like texture of the hydroalcoholic gel, one or more thickeners or gelling agents are present in a total amount of 0.4 - 2 % (by weight). Preferred thickeners/gelling agent(s) is/are selected from acrylate cross polymers, in particular C10-C30 alkyl acrylate cross polymers (such as commonly marketed under the tradename Carbopol^{®}), hydroxyethyl cellulose, xanthan gum and/or any combinations thereof. The amount of thickener mentioned is sufficient to ensure that the gel does not run off during application. In some embodiments, the hydroalcoholic gel comprises a thickener and/or gelling agent selected from acrylate cross polymers, hydroxyethyl cellulose, xanthan gum and/or any combinations thereof.

A hydroalcoholic gel may also comprise one or more pharmaceutically acceptable adjuvants, such as one or more of antioxidant(s), emulsifier (s), pH regulating agent(s), such as acid(s), base(s) or salt(s) thereof, stabilizer(s), colorant(s), or any combination thereof.

Further one or more emulsifiers may be used to provide an emulsion of the water phase and the oily substances present in the gel. A suitable emulsifier is e.g. sodium stearoyl glutamate (marketed as e.g. Carbopol ^{®}). The emulsifier is preferably present in a total amount of 0.01- 0.03 % (by weight). Examples of such suitable sodium stearoyl glutamate emulsifiers are marked under the trade name Carbopol 980, Carbopol 974p or Carbopol 9409.

The hydroalcoholic gel contains balance water in a quantity for the composition to obtain a total of 100%. Unless indicated otherwise, water is of drinking water quality, MilliQ water (e.g. purified water with a resistivity of at least 18.2 MΩ.cm (at 25°C) and a TOC value below 5 ppb.), or distilled water.

The hydroalcoholic gel may preferably further comprise 10-25% (by weight) of sodium chloride, in particular sea salt, or more preferred Dead Sea salt. The presence of sodium chloride in the hydroalcoholic gel has a positive effect in relation to psoriasis as the sodium chloride assists in lingering pruritus and/or in removing dead skin cells from the skin surface. In some embodiments, the composition comprises 10-15, 15-20, or 20-25% (by weight) sodium chloride, such as Dead Sea salt.

The composition may preferably further comprise one or more skin care or skin hydrating agents/moisturizers, selected from
i. 0.01-1 % (by weight) extract of aloe barbadensis leafs,
ii. 1-5 % (by weight) of panthenol,
iii. 0.1-1-5 % (by weight) of retinyl palmitate,
iv. 0.5-5 % (by weight) of glycerine,
or any combinations thereof, as the application of skin moisturizers to psoriasis prone skin further soothes the psoriasis prone skin and reduces the symptoms, in particular redness scaly skin and/or itch/pruritus. The moisturizers may also counteract any drying of the skin resulting from the application of ethanol (and thus potential removal of protective fat layers on the skin) present in the hydroalcoholic gel. Further, it appears as if cannabidiol boosts the effect of the above mentioned one or more moisturizers or vice versa, and thus may in fact provide a synergy in the regeneration and/or rehydration/moisturising of the skin where the composition is applied.

Unless indicated otherwise, "aloe barbadensis leafs" refers to freeze-dried leaves, usually in form of a powder.

Preferred amounts of the one or more skin care or skin hydrating agents/moisturizersn are listed below:
i. 0.01-0.5 % (by weight) extract of aloe barbadensis leafs, or more preferred 0.01-0.2 % (by weight)
ii. 1-5 % (by weight) of panthenol, or more preferred 2-3 % (by weight)
iii. 0.1-1 % (by weight) of retinyl palmitate, or more preferred 0.1-0,5 % (by weight),
iv. 0.5-5 % (by weight) of glycerine, or more preferred 0.5-2.5 % (by weight).

The topical composition may preferably further comprise one or more further components selected from 0.5-5 % (by weight) of menthol and/or 0.1-2 % (by weight) of camphor. Preferably menthol is present in amounts of 1-4 % by weight or more preferred 1.5-3 % (by weight). Camphor is preferably present in amounts of 0.2-1.0 % (by weight) or more preferred 0.4-0.8 % by weight. Further, eucalyptus oil may be added, preferably in an amount of 0.5-1.5% (by weight), or more preferred 0.4-0.8 % by weight, to further enhance skin penetration of the highly lipophilic cannabidiol.

Menthol camphor and/or eucalyptus oil provide a cooling feeling when applied to the skin, which may alleviate or reduce level of pain in joints suffering from rheumatism. Further, camphor, menthol and/or eucalyptus oil also act as penetration enhancers and to even further improve skin penetration of cannabidiol. In some embodiments, the hydroalcoholic gel comprises 0.5-5 % (by weight) menthol, 0.1-2 % (by weight) camphor and/or 0.5-1.5% (by weight) eucalyptus oil.

The topical composition may preferably further comprise one or more pharmaceutically acceptable adjuvants selected from antioxidants, pH regulating agents, such as acids or bases, stabilizers, colorants or any combination thereof.

A preferred antioxidant is Vitamin E (tocopherols) or one or more derivatives thereof, articular tocopheryl acetate (CAS no: 58-95-7). Antioxidants, such as tocopheryl acetate, may be present in a total amount of 1-3 % (by weight). Further, tocopherol acetate is known to provide improved wound healing and reduces formation of scar tissue when applied topically, which will be highly beneficial in relation to the treatment or reduction of symptoms from psoriasis.

Examples of other suitable antioxidants may include citric acid, ascorbic acid and/or combinations thereof. The latter also act as pH regulators. Phosphoric acid may also be applied as pH regulator and may ensure stable pH in the hydroalcoholic gel as it provides a phosphate buffer system in the hydroalcoholic gel.

For example, phytic acid (CAS. No: 83-86-3) may be added. Phytic acid can liberate phosphate ions and is thus able to provide pH stability due to creation of a phosphate buffering effect in the gel.

Alkaline pH regulators include commonly used water soluble and non-toxic bases, such as e.g. sodium hydroxide or potassium hydroxide.

Preferably, pH in the hydroalcoholic gel is 5-9, or preferably 6-8.5. In some embodiments, the pH of the gel mimics the pH of the skin, such as a slightly acidic pH. Thus in some embodiments, the pH is 4.5-6.5. In some embodiments, the pH of the hydroalcoholic gel is 4.5-5.5, 5.0-6.0, 5.5-6.5, 6.0-7.0, 6.5-7.5, 7.0-8.0, 7.5-8.5, or 8.0-9.0.

The cannabidiol used in preparation of the hydroalcoholic gel can be crystalline powder. The crystalline cannabidiol powder is obtained from natural cannabis plants and has a very high purity and very low or even extremely low levels of residual traces of other naturally occurring cannabinoids, in particular the cannabinoids with psychoactive effects, such as THC, as the systemic psychoactive effects is an unwanted side effects of the local administration of the gel. Crystalline cannabidiol has a very low level of traces of other cannabinoids, which is less than 2% (by weight) in total, and comprises less than 0.1 % (by weight) of THC.

In some embodiments, crystalline CBD can be characterized as follows (Table B):

**Table B: some characteristics of crystalline CBD (enecta^{®} product specification sheet**

| **TEST** | **SPECIFICATION** |
|---|---|
| Identification (NMR) | Positive |
| Purity (HPLC) | ≥ 98.0 % |
| Related substances (Purity HPLC): | |
| CBDV | ≤ 1.5 % |
| CBDA | ≤ 1.5% |
| CBG | ≤ 1.5 % |
| CBN | ≤ 1.5 % |
| THC | ≤ 0.10 % |
| Water content (KF) | ≤ 1.0% |
| Specific Rotation | -135.0 ± 5.0° |
| Melting point | 66.0 ± 3.0°C |
| Total ash | ≤ 0.30 % |

| | |
|---|---|
| *CBDV= Cannabidivarin (also called Cannabidivarol), CBDA = Cannabidiolic acid,* *CBG = Cannabigerol, CBN = Cannabinol, THC = Tetrahydrocannabinol* Cannabidiol (CBD) has been assigned CAS number 13956-29-1. Its empirical formula is C₂₁H₃₀O2. The chemical structure of CBD is given below: | |

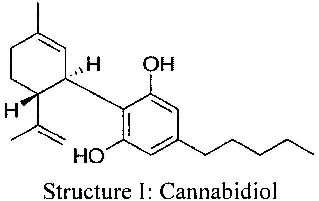

Surprisingly and unexpectedly, the inventors have found that the use of CBD with a high degree of purity, such as "crystalline CBD", provides one or more advantages in the formulation and use of a hydroalcoholic gel as disclosed herein. Firstly, CBD is usually provided dissolved in a carrier oil, such as a vegetable oil, e.g. coconut, or hemp seed oil. Commonly, such a "CBD oil" comprise CBD in a concentration of about 10% (by weight). The use of CBD oil thus results in formulations with a significant amount of (vegetable) oil. For example, a gel formulated with a CBD concentration of 1 % will comprise 10% oil. In some embodiments, the use and/or presence of oil, such as vegetable oil(s) is not desired in the formulation, as the oil may even lead to allergic reactions. Without wanting to be bound by any theory, it appears surprising, that formulations comprising vegetable oil are less efficient than formulations without vegetable oil as disclosed herein, in view of many vegetable oil comprising topical compositions, including ointments, e.g. pain relieving ointments. Secondly, providing CBD from CBD oil resulted in the need for significantly higher CBD doses, compared to a pure CBD, such as crystalline CBD, in order to provide a similar and/or comparable effect. This is very surprising, as prior art teaches that the presence of further active components in more crude CBD formulations such as in CBD oils provides beneficial, synergistic effects.

In some embodiments, at least 10, 20, 50% or even 100% or more CBD from CBD oil is needed to provide a similar effect when compared to formulations with pure, crystalline CBD.

In some embodiments, Hydroalcoholic gel composition comprises no vegetable and/or mineral oil. In some embodiments, the composition comprises less than 5, 1, or 0.1% (by weight) oil, such as vegetable and/or mineral oil.

In some embodiments, the CBD is "pure" or "crystalline" CBD, such as CBD composition comprising less than 1.5% (by weight) of any one of: Cannabidivarin (CBDV), Cannabidiolic acid (CBDA), Cannabigerol (CBG), Cannabinol (CBN); and/or less than 1.0% (by weight) of Tetrahydrocannabinol (THC). In some embodiments, the "pure" or "crystalline" CBD comprises less than 1.0% (by weight) of any one of: CBDV, CBDA, CBG, CBN; and/or THC. In some embodiments, the "pure" or "crystalline" CBD comprises less than 0.50, 0.2, or 0.1% (by weight) of any one of: CBDV, CBDA, CBG, CBN; and/or THC. In some embodiments none of CBDV, CBDA, CBG, and CBN is present in a concentration of more than 1.5 % by weight, and THC in more than 1% (by weight). In some embodiments, none of CBDV, CBDA, CBG, CBN and THC is present in a concentration of than 1.0, 0.5, 0.2 or 0.1 % by weight,

The composition is also applicable for use as a cosmetical skin care product that provides a hydrating/moisturizing effect on the epidermis. Further, cannabidiol's properties as an antibacterial agent may also protect the skin surface from mild infections.

Composition(s) described herein may also be suitable to treat, reduce and/or alleviate symptoms from psoriasis. Generally, the severity of psoriasis can be divided into "mild", i.e. below 3% of skin surface has psoriasis; "moderate", 3 - 10% of skin surface has psoriasis, and "severe" above 10% of the skin surface has psoriasis.

In some embodiments, a hydroalcoholic gel composition is suitable for treatment, reduction and/or alleviation of mild psoriasis symptoms.

In some embodiments, a hydroalcoholic gel composition is suitable for treatment, reduction and/or alleviation of moderate psoriasis symptoms.

In some embodiments, a hydroalcoholic gel composition is suitable for treatment, reduction and/or alleviation of severe psoriasis symptoms.

In some embodiments, a hydroalcoholic gel composition is suitable for treatment, reduction and/or alleviation of mild, moderate, and/or severe psoriasis symptoms, i.e. any combination of mild, moderate, and/or severe psoriasis symptoms.

A hydroalcoholic gel composition for local topical application in order to reduce or alleviate symptoms from psoriasis can also be referred to as "psoriasis gel" herein.

A hydroalcoholic gel composition for local topical application in order to reduce or alleviate symptoms from psoriasis may e.g. comprise salt, such as sea salt, e.g Dead Sea salt, ethanol, water, panthenol, tocopheryl acetate, hydroxycellulose, cannabidiol, isopropyl myristate, retinyl palmitate, Glycerin F, Aloe barbadensis leaf, and citric acid.

In some embodiments, a psoriasis gel comprises: Dead Sea Salt, Glycerin F, Ethanol denat 96%, Isopropyl Myristate, Aloe Barbadensis Leaf, Water, Panthenol, Hydroxyethylcellulose, Cannabidiol CBD, Xanthan, Retinyl Palmitate, Tocopheryl Acetate and optionally sodium hydroxide, e.g. aqueous sodium hydroxide solution.

Further examples of psoriasis gels suitable for treatment/reduction and/or alleviation of psoriasis are given above and/or below, including suitable ranges of the respective ingredients.

A more preferred hydroalcoholic gel composition that is particularly prepared for local topical application in order to reduce or alleviate symptoms from psoriasis comprises, consists essentially of, and/or can be provided by combining:
0.1-20 % (by weight) or more preferred 0.1-10 % (by weight) of cannabidiol,
0.5-1.5 % (by weight) of a skin penetration enhancer, in particular isopropyl myristate,
0.5-1.5 % (by weight) of glycerine,
10-30 % (by weight) of ethanol,
0.4-2 % (by weight) in total of one or more thickeners or gelling agents, as mentioned above,
12-18 % (by weight) of sodium chloride, in particular sea salt, or more preferred Dead Sea salt,
0.01-1 % (by weight) of extract of aloe barbadensis leafs,
1-5 % (by weight) of panthenol,
0.1-1.5 % (by weight) of retinyl palmitate,
0.5-5 % (by weight) of glycerine, and
water in a quantity for the composition to a total of 100%.

A further hydroalcoholic gel composition that is particularly prepared for local topical application in order to reduce or alleviate symptoms from psoriasis may consist essentially of, it may comprise, and/or it can be provided by combining:
0.5-2.0 % (by weight) or more preferred ~1.0 % (by weight) of cannabidiol,
0.3-1.5 % (by weight) or more preferred ~0.9 % (by weight) of a skin penetration enhancer, in particular isopropyl myristate,
0.1-0.5 % (by weight) or more preferred ~0.2 % (by weight) of glycerine,
10-30 % (by weight) of ethanol or more preferred ~15 % (by weight) of ethanol, in particular 96% ethanol, such as 96% denatured ethanol,
0.5-2.5 % (by weight) or more preferred ~1.5 % (by weight) of one or more thickeners or gelling agents, in particular hydroxyethyl cellulose,
10-20 % (by weight) or more preferred ~15.0 % (by weight) of sodium chloride, in particular sea salt, or more preferred Dead Sea salt,
0.01-1 % (by weight) or more preferred -0.05 % (by weight) of extract of aloe barbadensis leafs,
1.5-4.0 % (by weight) or more preferred -2.75 % (by weight) of panthenol,
0.1-0.5 % (by weight) or more preferred -0.3 % (by weight) of retinyl palmitate,
0.1-0.3 % (by weight) or more preferred -0.2 % (by weight) of glycerine,
0-01-0-03 % (by weight) or more preferred -0.02 % (by weight) of an antioxidant,
such as citric acid, and in particular citric acid monohydrate, and
water in a quantity for the composition to a total of 100% (by weight), such as 75.88 - 53.83 % (by weight), more preferred ~61.28 % (by weight), said water being preferably water of drinking water quality.

The psoriasis gel causes proliferation activity inhibition. Since psoriasis is an inflammatory skin disease which produces an excessive hyperproliferation of keratinocytes, the test substance could be beneficial for the treatment of this disease as shown in the examples given below.

A highly preferred hydroalcoholic gel composition that is particularly prepared for local topical application in order to reduce or alleviate local symptoms, e.g. pain, resulting from rheumatism in one or more joints, and or reducing inflammation level in the one or more joints and/or reducing neurological pain, such as pain resulting from sclerosis, e.g. multiple sclerosis, comprises
0.1-10% (by weight) cannabidiol, or more preferred 0.-2.0 % (by weight) of cannabidiol,
0.5-1.5% (by weight) of a skin penetration enhancer, in particular isopropyl myristate, 20-30 % (by weight) ethanol,
0.5-1.5 % (by weight) in total of or more preferred 0.8-1.2 % (by weight) in total of one or more thickeners or gelling agents, as mentioned above,
1.5-2.5 % (by weight) menthol,
0.4-0.7 % (by weight) of camphor, and
water in a quantity for the composition to a total of 100%.

A highly preferred hydroalcoholic gel composition that is particularly prepared for local topical application in order to reduce or alleviate symptoms from psoriasis comprises
0.1-10% (by weight) or more preferred 0.5-2.0 % (by weight) of cannabidiol,
0.5-1.5% (by weight) of a skin penetration enhancer, in particular isopropyl myristate,
0.5-1.5 % (by weight) of glycerine,
10-20 % (by weight) of ethanol,
0.4-2 % (by weight) in total of one or more thickeners or gelling agents, as mentioned above,
12-18 % (by weight) of sodium chloride, in particular sea salt, or more preferred Dead Sea salt,
0.01-1 % (by weight) of extract of aloe barbadensis leafs,
2-3 % (by weight) of panthenol,
0.1-0.5 % (by weight) of retinyl palmitate,
1-5 % (by weight) of glycerine, and
water in a quantity for the composition to a total of 100%.

Without wanting to be bound by any theory, it is believed that e psoriasis gel causes proliferation activity inhibition. Since psoriasis is an inflammatory skin disease which produces an excessive hyperproliferation of keratinocytes, the test substance could be beneficial for the treatment of this disease as shown in the examples given below.

Treatment of psoriasis, such as mild, moderate and/or severe psoriasis may comprise:
Application of the psoriasis on the surface of the skin by gently massaging a suitable amount of the gel onto the parts of the body to be treated (e.g. 0,25 g -size of a pea to hand, wrists or elbows (∼25 cm²), or 0.5 g size of 2 peas to foot, ankle or knee (-50 cm²).

Dosage: 1 -5 times per day, preferably 1-3 times per day, more preferably 1-2 times per day.

In some embodiments, the gel is washed of the skin surface after a certain amount of time. It is believed that certain skin-affine cosmetic substances, as skin care products may remain.

After around 30 min, 45 or an hour at the latest, the skin is rinsed or washed with water, such as warm tap water, primarily to remove excess salt.

A hydroalcoholic gel composition for local topical application in order to reduce or alleviate symptoms e.g. pain, resulting from rheumatism in one or more joints, and/or reducing inflammation level in the one or more joints can also be referred to as "arthirits gel" herein.

A most preferred hydroalcoholic gel composition that is particularly prepared for local topical application in order to reduce or alleviate local symptoms, e.g. pain, resulting from rheumatism in one or more joints, and/or reducing inflammation level in the one or more joints comprises
0.2-5 % (by weight) or more preferred 0.2-2 % (by weight) cannabidiol,
0.7-1.1 % (by weight) of a skin penetration enhancer, in particular isopropyl myristate, 20-30 % (by weight) ethanol,
0.5-1.5 % (by weight) in total of or more preferred 0.8-1.2 % (by weight) in total of one or more thickeners or gelling agents, as mentioned above,
1.5-2.5 % (by weight) menthol,
0.4-0.7 % (by weight) of camphor,
0.05-0.3 % (by weight) Phytic acid (of 50 % purity), and
water in a quantity for the composition to a total of 100%.

A most preferred hydroalcoholic gel composition that is particularly prepared for local topical application in order to reduce or alleviate symptoms from psoriasis comprises
0.2-5% (by weight) or more preferred 0.2-2.0 % (by weight) of cannabidiol,
0.7-1.1 % (by weight) of a skin penetration enhancer, in particular isopropyl myristate, 0.5-1.5 % (by weight) of glycerine,
10-20 % (by weight) of ethanol,
0.4-2 % (by weight) in total of one or more thickeners or gelling agents, as mentioned above,
12-18 % (by weight) of sodium chloride, in particular sea salt, or more preferred Dead Sea salt,
0.01-0.1 % (by weight) of extract of aloe barbadensis leafs,
2-3 % (by weight) of panthenol (of 75 % purity),
1.0-3.0 % (by weight) of tocopherol acetate
0.1-0.5 % (by weight) of retinyl palmitate,
1-3 % (by weight) of glycerine,
0.01-0.03 % (by weight) sodium stearoyl glutamate ( an O/W emulsifier)
0.01-0.03 % (by weight) of citric acid (monohydrate), and
water in a quantity for the composition to a total of 100%.

The psoriasis gel causes proliferation activity inhibition. Since psoriasis is an inflammatory skin disease which produces an excessive hyperproliferation of keratinocytes, the test substance could be beneficial for the treatment of this disease as shown in the examples given below.

The compositions described above are applicable for use as a medical composition in a local topical application in the treatment or alleviation of symptoms, in particular pain, resulting from arthritis, in particular rheumatoid arthritis, osteoarthritis, juvenile rheumatoid arthritis and/or psoriatric arthritis in a subject, such as a humans, mammal or other animal, such as cats, dogs or horses.

In some embodiments, the compositions described above are applicable for use as a medical composition in local topical application in the treatment or alleviation of symptoms of psoriasis, in particular of red, dry, itchy, and/or scaly skin, in a subject, such as a human, mammal or other animal.

The topical gel composition is applied topically to a local area of the epidermis of a subject, such as a human, mammal, or other animal 1-5 times daily. In some embodiments, the gel composition is applied once or twice per day. In some embodiments, the composition is applied 3, 4 or more times per day. Suitable dosages for a subject, such as a human, are e.g. 0.25 g (approximately the size of a pea) to hand, wrists or elbows, such as an area of around 25 cm², or apply 0.5 g size of 2 peas to foot, ankle or knee (e.g. 50 cm²).

In some embodiments, a gel composition as disclosed herein is applied topically to a local skin area of a subject in an amount of 2-250, 5-100, or 10-50 mg/ cm² per application.

In some embodiments, one or more topical gel composition(s) as disclosed herein are applied to alleviate a painful condition associated with arthritis and/or one or more hot and/or swollen joints.

Generally, compositions as disclosed herein unless indicated otherwise are topical compositions.

Compositions as disclosed herein can also be cosmetic compositions. In some embodiments, the composition is a cosmetic skin hydrating or skin care composition.

Generally, it is believed that the presence of alcohol in a composition according to the present invention may provide a beneficiary effect for the subject through rapid evaporation condoning a cooling effect on the treated skin surface.

Examples for instructions for use of gels disclosed herein are given in Example 11 and 12.

In some embodiments, a composition as disclosed herein is part of a kit, comprising an instruction for use, such as an instruction for use disclosed in Example 11 or 12. Often, the composition is packaged in a suitable container, such as a container that can be opened and closed repeatedly or a single use container. In some embodiments, the container is a single use container, such as a sealed bag. In some embodiments, the container is a glass or plastic containers with a lid. In some embodiments, the container is a tube, such as a squeeze and/or collapsible, or another collapsible package known in the art. In some embodiments, a composition as disclosed herein is provided in a container, such as a kit, which may also comprise an instruction for use.

Further embodiments of the present invention are also disclosed in the following Examples and Figures.

### Description of the Drawing

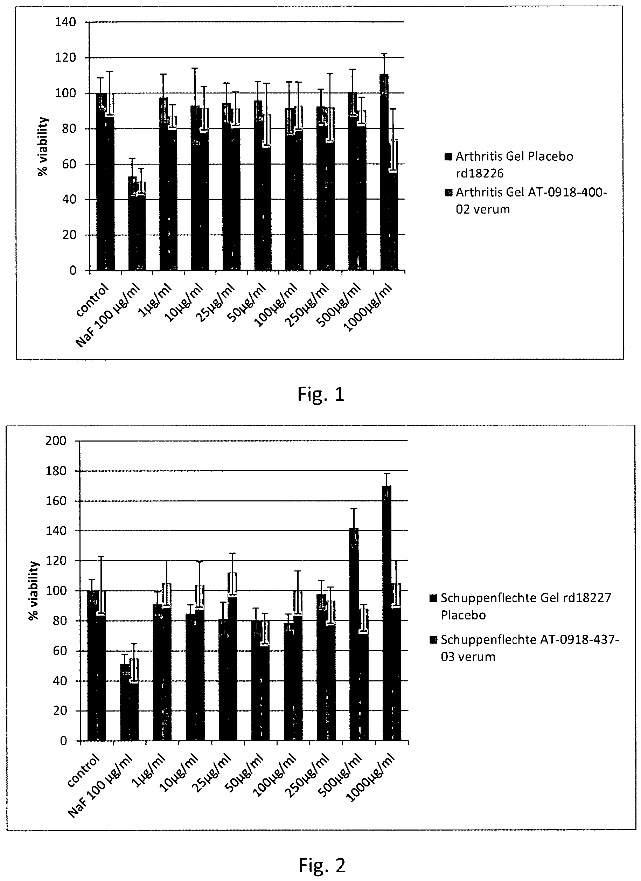

Figs. 1-7 show graphics of results from Examples 1-7 below.

### Examples

### Abbreviations

NaF: natrium fluoride
LPS: lipopolysaccharide
IL: interleukine
TNF: tumor necrosis factor
PGE2: Prostaglandin E2
r.l.u.: relative light units
SD: standard deviation
DMEM: Dulbecco's Modified Eagle Medium
FBS: Fetal bovine serum
EF-1α: Elongation factor 1α

### Example 1 - Preparation of psoriasis hydroalcoholic gel and placebo psoriasis gel for in vitro tests

Psoriasis gel and a placebo gel were produced with the following compositions:

**Table 1. Composition of psoriasis gel and corresponding placebo gel**

| Ingredient | Producer/origin or grade | Psoriasis gel (verum) Amount in g in a total of 100 g (or w/w %) | Placebo Psoriasis gel Amount in g in a total of 100 g (or w/w %) |
|---|---|---|---|
| Water | demineralized | 55.2 | 92.07 |
| Ethanol | DICKE/96% (vol) Pharma grade | 15.00 | - |
| Cannabidiol CBD | Enecta/>=98% pure CBD crystalline powder, | 2.00 | - |
| Aloe Barbadensis leaf extract (aqueous) | Terry Laboratories/TERRA-PURE Non-preserved spray dried Aloe vera powder 200X inner leaf/USP & FCC | 0.05 | 0.05 |
| Dead Sea salt | Krüger Gourmet/Totes Meer Salz/FCC | 20.00 | - |
| Panthenol | DICKE/D-Panthenol 75% FCC grade | 2.75 | 2.75 |
| Tocopheryl acetate | Zhejiang Medicine Co. Ltd., Xinchang Pharmaceutical Factory, China/ DL-alpha Tocopheryl Acetate/EP grade | 2.00 | 2.00 |
| Retinyl Palmitate | Kyowa Hakko Europe GmbH /Vitamin A Palmitate 1.0M /FCC & USP grade | 0.3 | 0.3 |
| Isopropyl myristate | Pionier IPM/- Isopropyl Myristat/ EP grade | 0.90 | 0.90 |
| Hydroxyethyl cellulose | ShinEtsu/Tylose H 100000 YP2 | 1.13 | 1.13 |
| Glycerine, Glycerol | BASF/Eumulgin VL 75 | 1.00 | - |
| Xanthan gum | CP Kelco/ Keltrol CG-BT/Food grade | 0.6 | 0.6 |
| NaOH | Azelis/Sodium Hydroxide pellets/ EP grade | 0.07 | - |
| Potassium Sorbate | Merck/Potassium sorbate granules EMPROVEexp/EP, BP, FCC E202 | - | 0.2 |
| Sum | | 100 g | 100 g |

Ethanol was mixed with cannabidiol, glycerine and isopropyl myristate at room temperature.

Water was mixed with the remaining ingredients also at room temperature. Then, the ethanolic fraction was poured into the aqueous fraction and stirred until a homogenous mixture was obtained. The thickener is added to the water fraction before mixing with the ethanolic fraction and/or to the mixture of ethanolic and water fractions.

For the placebo gel, all ingredients were thoroughly mixed at room temperature.

### Example 2 - Preparation of arthritis hydroalcoholic gel and placebo arthritis gel for in vitro tests.

Arthritis gel and a placebo arthritis gel were produced as described in example 1 with the following compositions:

**Table 2. Composition of arthritis gel and corresponding placebo gel**

| Ingredient | Producer/origin/ grade/trade name | Arthritis gel (verum) Amount in w/w % | Placebo Arthritis gel Amount in w/w % |
|---|---|---|---|
| Water | demineralized | 68.34 | 97.9 |
| Ethanol | DICKE/96% (vol) Pharma grade | 25.00 | - |
| Cannabidiol CBD | Enecta/>=98% pure CBD crystalline powder | 1.00 | - |
| Menthol | DULLBERG/L-menthol natural/EP & USP grade | 2.00 | - |
| Camphor | Frey+Lau/Campher synth. White crystalline powder | 0.56 | - |
| Isopropyl myristate | Pionier IPM/ Isopropyl Myristat/ EP grade | 0.9 | 0.9 |
| Acrylate Cross polymer | Lubrizol/Carbopol^{®} Ultrez 20 Polymer | 1.0 | 1.0 |
| NaOH | Azelis/Sodium Hydroxide pellets/ EP grade | 1.30 | - |
| Potassium Sorbate | Merck/Potassium sorbate granules EMPROVEexp/EP, BP, FCC E202 | - | 0.2 |
| Sum | | 100 g | 100 g |

### Example 3 - Arthritis gel's/placebo's effect on viability of human monocytes

### Monocyte cell cultures

Human primary monocytes were prepared from buffy coats of healthy human blood donors following a standardized procedure.

### Monocyte cell treatment and Alamar-Blue-assay

Cells were seeded in 96-well-plates at a density of 220,000 cells/well for viability measurements.

Arthritis gel and placebo arthritis gel (test items made in example 2), respectively, were dissolved in cell culture media. 1 µl of the stock solution or the dilutions were added per well (100 µl). Monocytes were seeded in 96 wells and incubated with: NaF (100 µg/ml) positive control, media control and increasing concentrations of the test items.

Monocytes were seeded in 96 wells and incubated with 8 different concentrations of the test items.

NaF was used as positive and non-treated cells as negative control. Tested concentrations of test items appear in fig. 1.

After 24 h of incubation 10 µl AlamarBlue^{®} (Biosource, USA) was added to each well. After 2 hours the fluorescence was measured with a fluorescence spectrophotometer using 544EX nm/590EM nm filter settings. The amount of fluorescence is proportional to the number of living cells and corresponds to the cells metabolic activity. Damaged and nonviable cells have lower innate metabolic activity and thus generate a proportionally lower signal than healthy cells.

The active ingredient of alamarBlue^{®} (resazurin) is a nontoxic, cell permeable compound that is blue in color and virtually nonfluorescent. Upon entering cells, resazurin is reduced to resorufin, which produces very bright red fluorescence. Viable cells continuously convert resazurin to resorufin, thereby generating a quantitative measure of viability and cytotoxicity.

The effect on cell viability in human monocytes is shown in fig. 1. Results are expressed as % viability compared to control (100%) (mean: n=4 ± standard deviation).

Up to 500 µg/ml none of the test items, i.e. neither the arthritis gel nor the placebo arthritis gel, were cytotoxic.

### Example 4 - Psoriasis gel's/placebo's effect on viability of human monocytes

Test in example 3 was repeated for the test items prepared in example 1 to test cyto-toxity of psoriasis gel and placebo psoriasis gel on human monocytes.

The results on the effect on cell viability in human monocytes are shown in fig. 2. Results are expressed as % viability compared to control (mean: n=4 ± standard deviation).

Up to 1000 µg/ml none of the test items was cytotoxic.

At concentrations ≥ 500 µg/ml placebo showed "higher viability" which might be due to interference with fluorescence measurement.

### Example 5 - Effects of arthritis gel on LPS induced IL-6 release and PGE₂ release

Human primary monocytes were isolated from buffy coats of 3 healthy human blood donors.

Cells were seeded in 24-well plates at a density of 2,200,000 cells/well for ELISA measurements.

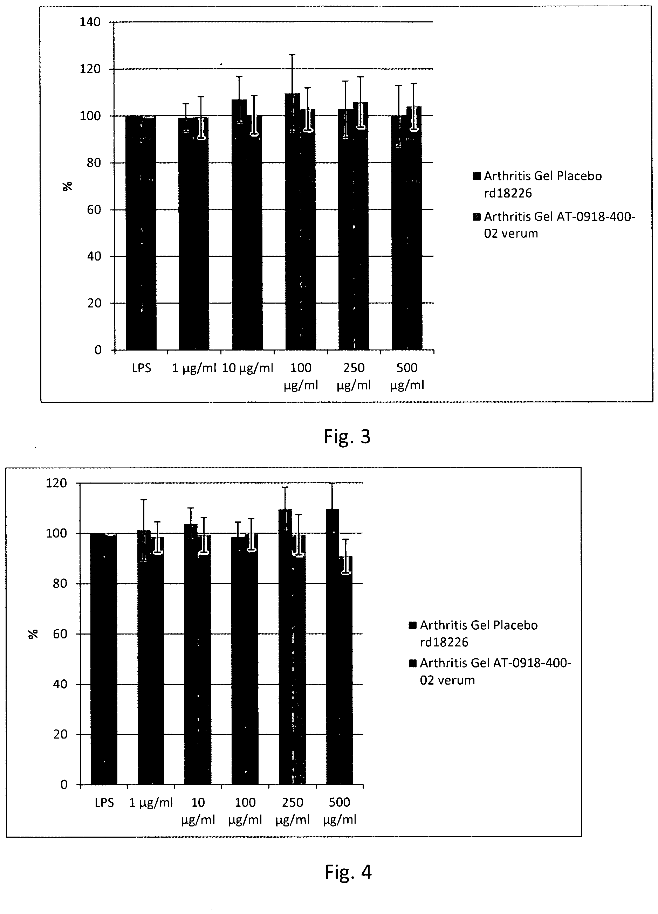

Monocytes were incubated with the test items prepared in example 2 (arthritis gel and placebo gel, respectively (5 different concentrations, the used concentrations appear in fig. 3.) for 24 h.

LPS (10 ng/ml, salmonella typhimurium SL1181) was added 30 min after start of the treatment with the test items.

After 24 h supernatants were removed, centrifuged and investigated for IL-6 (fig. 3) concentrations in ELISA using manufacturer's protocol (n=6, 3 different donors).

The results shown in fig. 3 are expressed as % of LPS value (LPS = 100 %, mean: n=6 ± standard deviation).

Both test items (placebo and arthritis verum) had no effect on LPS induced IL-6 release in human monocytes.

Effects on LPS stimulated PGE2- release is shown in fig. 4.

Results are expressed as % of LPS value (LPS = 100 %, mean: n=6 ± standard deviation).

At the highest concentration tested, the arthritis gel (verum) inhibited the LPS induced PGE2 release whereas the arthritis placebo gel slightly enhanced the LPS induced PGE2 release.

### Example 6 - Effects of psoriasis gel on LPS induced TNF release

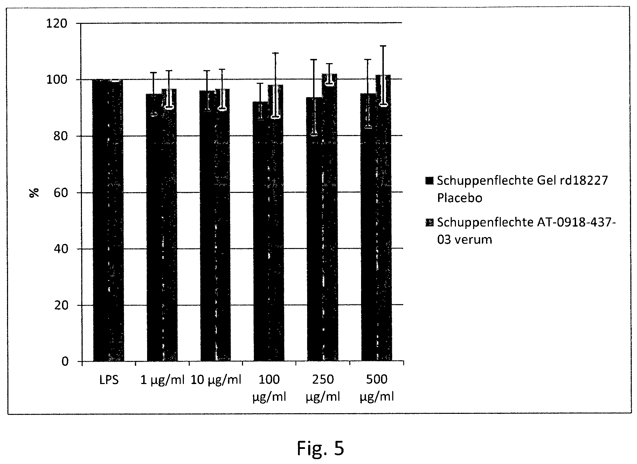

Human monocytes of three different donors were stimulated with LPS (10 ng/ml) and incubated with increasing concentrations of the test items made in example 1, i.e. the psoriasis gel and the placebo psoriasis gel. The used concentrations of the test items appear in fig. 5. Results are expressed as % of LPS value (LPS = 100 %, mean: n=6 ± standard deviation).

Both test items (psoriasis gel (verum) and placebo) had no effect on LPS induced TNFα release.

### Example 7 - Effect of psoriasis gel on proliferation of keratinocytes.

The psoriasis gel (PG) and the placebo psoriasis gel (PPG) were tested for their effect on proliferation of keratinocytes.

The following concentrations were investigated with respect to the effects on proliferation: 10, 25, 50, 100, 250, 500, 1000 µg/ml.

Keratinocytes cell cultures:
The cell lines HaCaT and HaCaT-NucLight-Red (keratinocytes) were cultured in supplemented DMEM medium containing 10% FBS and 1% antibiotics penicillin/streptomycin (DMEM complete medium) at 37°C in a humidified atmosphere of 5% CO₂. HaCaT-NucLight-Red cells were generated in our lab by infection with a lentivirus encoding a nuclear restricted Red Fluorescent Protein under the EF-1α promoter.

### Proliferation assays

The IncuCyte^{™} Live-Cell Imaging Systems in conjunction with NucLight reagents provides a live cell, kinetic assay for the measurement of proliferation (Essen BioScience). HaCaT-NucLight-Red cells were seeded at a density of 5×10³ cells/well in 96-well plates at 37°C in a humidified atmosphere of 5% CO₂. Then, cell cultures were treated with the selected concentrations of the test substances in DMEM medium containing FBS 10%, the plates were introduced in an IncuCyte ZOOM live cell microscope (Essen BioScience), and images were taken every 24 hours for an additional 72 hours. The data were analyzed by the total integrated intensity (RCUxµm²×Well) of the live content cell imaging system IncuCyte HD (Essen BioScience, Hertfordshire, UK). The assays for each concentration of the test items were done in triplicate wells.

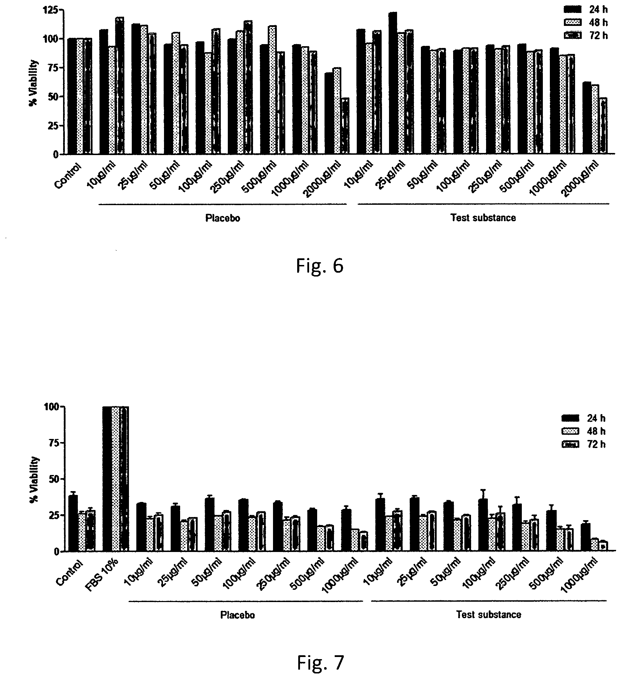

HaCaT-NucLight-Red cells were treated with test substance or placebo at doses indicated for 24, 48 and 72 h. Proliferation activity was evaluated by IncuCyte ZOOM Live-Cell Imaging System. Results are shown in fig. 7.

The test substance decreases proliferation compared to placebo at a dose of 1000 µg/ml after 24, 48 and 72 h as can be seen in fig. 7. No significant changes have been found for the remaining doses (Fig. 7).

### Example 8 - Human test of psoriasis gel

Four test persons (all females) accepted to test the psoriasis gel (verum/placebo) prepared according to example 1.

All four had active psoriasis spots as indicated in table 3 below. None of the test persons took any other medication during test period.

Each test person was requested to rate symptoms on a 0-10 scale ( 0= no symptoms, 10=worst ever experienced symptoms of psoriasis) before testing gel as well as during the test, i.e. 1 hour after applying the gel to the psoriasis spots mentioned in table 3 and as an average over the day. - Similar after testing the gel for the number of days mentioned in table 3.

Each test person applied the psoriasis gel (verum or placebo) over the specified spots twice daily during the test period (period mentioned in table 3).

Test person 1, 2 and 4 reported that a salt layer appeared about 1 hour after application of the gel. The salt layer was easily removed with a wetted cloth.

Test persons 1, 2 and 4 all reported that the itchiness disappeared within 10-20 minutes after application. Test persons 1 and 4 reported experiencing a sensation of heat shortly after application of the gel.

**Table 3. Psoriasis gel test**

| | Test person 1 (Verum) | Test person 2 (Verum) | Test person 3 (Placebo) | Test person 4 (Verum) |
|---|---|---|---|---|
| Psoriasis spot size and position | 12×1 cm red, scaling and very ichy spot at hair line ( forehead) | 2x2 cm red, scaling, ichy spot on right elbow | 2 spots of 1x1 cm on top of right hand | 2x1 cm spot between right eye and nose |
| Rate of symptoms (scale 1-10) | | | | |
| A: before test period | 8-9 | 8 | 6-7 | 9-10 |
| B: 1 hour after application | 6 | 4 | 6 | 6-7 |
| C: average during day in test period | 4 | 2 | 5-6 | 2-4 |
| D: after end of test | 0 | 0 | - | 0 |
| Test period ( days) | 2 | 14 | 30 | 14 |
| | The inflammation starts to disappear after the second day of application. | The redness is reduced gradually after second day of treatment. | Only slight reduction in itchy sensation and slight reduction in scaling occurred at end of test period. | and the redness is reduced gradually. |
| | The symptoms did not revert after ending treatment | | The red scaly spots did not reduce in size, nor did they increase in size. | |
| | | | | |

The test clearly indicates that the verum psoriasis gel reduces itchiness, scaling, redness and duration of psoriasis when applied twice daily. The placebo gel does not provide any significant reduction in symptoms of itchiness, scaling or redness.

### Example 9

Three test persons tested the Arthritis gel. Two test persons had symptoms of osteoarthritis from their right elbow, the third person had symptoms from the neck.

Each test person was requested to rate symptoms on a 0-10 scale ( 0= no symptoms, 10=worst ever experienced pain from arthritis) before testing gel as well as during the test, i.e. 1 hour after applying the gel to the elbows/neck as an average over the day. - Similar after testing the gel for the number of days.

None of the test persons took prescription medication against osteoarthritis. All three test persons took over the counter painkillers at beginning of the test period. Test person 1 and 2 took Ibuprofen 3 times daily (400 mg) and test person 3 took paracetamol 2×500 mg 4 times daily during the test period.

Each test person applied the arthritis gel (verum) on the mentioned joints twice daily during the test period (period mentioned in table 4) or as pain appeared.

All three test persons reported that there was slightly pain relief within minutes after application of the gel and after 1-2 hours the pain was close to zero.

**Table 4: Test Arthritis gel on pain caused by arthritis**

| | Test person 1 | Test person 2 | Test person 3 |
|---|---|---|---|
| Joint with symptoms of osteoarthritis | Right elbow | Right elbow | Neck |
| Rate of symptoms (scale 1-10) | | | |
| A:before test period | 10 ( without taking non-prescription medication) when taking | 5 | 7 |
| B: 1 hour after | | 0-1 | 1-2 |
| application | 1-2 | | |
| C: average during day in test period | | 1 | 0-1 |
| | 2 | | |
| | while taking Ibuprofene; after stopping taking Ibuprofen, pain slightly rose to 2-3 but slowly returned to 2 and then further reduced to 0-1 at end of test | | |
| D: after end of test | 0-1 | 0 | 1 |
| | | Pain did not appear again after ending application | |
| Test period ( days) | 30 | 5 | 30 |
| Medication reduced during test period? | Yes, after 11 days Ibuprofen dose was eliminated | Yes, at beginning of test period Ibuprofen dose was eliminated. | After 3 weeks paracetamol was eliminated. |

The test clearly indicates that the verum arthritis gel reduces pain from joints when applied twice daily.

### Example 10

Two test persons suffering from mild multiple sclerosis (MS) tested the Arthritis gel relative and the effect on local neurological pain caused by MS. Both test persons had pain caused by MS in their arms. One test person experienced MS related pain in the right arm only and one test person experienced MS related pain in both arms.

Each test person was requested to rate the neurological pain on a 0-10 scale (0= no symptoms, 10= worst ever experienced pain from arthritis) before testing gel as well as during the test, i.e. 1 hour after applying the gel to the right arm as an average over the day. - Similar after testing the gel for the number of days.

Both test persons took medication against MS, in both cases Rebif22^{®} (Interferon beta-1a (CHO-zellen, 22 mg), in both cases three times a week. None of the persons reduced the weekly dose thereof during the test. During periods with pain attacks both test person took ibuprofen 400 mg 3 times daily.

Each test person applied the arthritis gel (verum) on their right arm twice daily during the test period (period mentioned in table 5). Left arm of second test person was not treated. First test person suffered from MS attack in 14 days. Second test person suffered from MS attack in 60 days. Both test persons tested the arthritis gel for 14 days.

Both test persons reported that there was some pain relief within minutes after application of the gel, and after 2 hours the pain was close to zero in their right arm, while pain was not reduced in second test person's the left arm.

**Table 5: Test Arthritis gel against pain related to MS.**

| | Test person 1 | Test person 2 |
|---|---|---|
| Bodypart with symptoms of MS related pain | Right arm from above elbow | Right and left arm from above elbow; gel was applied only to right arm |
| Rate of symptoms (scale 1-10) | | |
| A:before test period | | |
| | 6 | 6 in both arms |
| B: 2 hours after application | | |
| | 2, lasting for about half a day | |
| | | Right arm: 2, lasting for about half a day |
| C: average during day in test period | | |
| | 3 | |
| | | Left arm: 6 all day |
| D: after end of test | 0; attack had ended at end of test period; pain did not reappear | |
| | | 1; |
| | | 0 |
| | | Pain did not appear again after ending application |
| Test period ( days) | 14 | 14 |
| Medication reduced during test period? | No | No |

### Conclusions on test results:

### Arthritis Gel Placebo and Arthritis Gel (verum):

At the highest concentration tested (500 µg/ml) Arthritis Gel AT-0918-400-02 verum inhibited the LPS induced PGE2 release, whereas placebo slightly enhanced the LPS induced PGE2 release.

Prostaglandin E2 (PGE-2) is usually released by blood vessel walls in response to infection or inflammation and acts on the brain to induce fever and/or sensing pain. Thus, by inhibiting the LPS induced PGE2 release, the verum arthritis gel may thus also assist in reducing the level of temperature increase and sensed pain in the locally treated area.

Further, Camphor as well as menthol is known to improve blood circulation in skin areas where camphor and/or menthol is applied topically. Thus, the combination of menthol, camphor and cannabidiol in the hydroethanolic gel appears to improve skin penetration of cannabidiol as well as to boost each other and promote relieving pain in the vicinity of the local, topical area treated with the arthritis gel.

The test by test persons in example 9 clearly indicates that the verum arthritis gel reduces pain from joints. The effect is seen when applied at least twice daily.

### Placebo Psoriasis Gel and Psoriasis gel (verum):

Both test items (placebo and verum) had no effect on LPS induced TNFα release.

The effect of the test items on the viability HaCaT was preliminary analysed. Up to 1000 µg/ml none of the test items was cytotoxic.

Therefore the following concentrations were investigated with respect to the effects on proliferation: 10, 25, 50, 100, 250, 500, 1000 µg/ml.

Psoriasis gel (verum) causes proliferation activity inhibition. However, it should be taken into account that these effects are also observed in the placebo although to a lesser extent than the verum. Since psoriasis is an inflammatory skin disease which produces an excessive hyperproliferation of keratinocytes, the test substance could be beneficial for the treatment of this disease.

Further, when tested on test persons in example 8, the test clearly indicates that the verum psoriasis gel reduces itchiness, scaling, redness and duration of psoriasis. The effect is seen when applied at least twice daily. The placebo gel does not provide any significant reduction in symptoms of itchiness, scaling or redness.

### Arthritis Gel (verum) effect on neurological pain from MS:

The test in example 10 clearly indicates that the verum arthritis gel reduces pain from attacks of MS. The effect is seen when applied at least twice daily.

### Example 11

### Instructions for use - Arthritis Gel

### How does Arthritis gel work?

Arthritis Gel is a gel preparation with an alcohol / water mixture, which
- reduces the painful conditions that occur in rheumatoid arthritis
- causes a conductive cooling effect

Arthritis gel is a medical device. Efficacy and safety have been tested according to the European Directive on Medical Devices (93/42 / EEC).

### Active principle of Arthritis gel:

Arthritis gel is applied externally in the form of a gel preparation in painful conditions of hot and swollen joints. It is used to support the external treatment of arthritis and reduce the painful conditions that occur in these patients. The main physical effect is caused by the alcohol/water mixture. When the gel is applied on the skin, the alcohol evaporates, causing a faster conductive cooling effect on the treated skin surface.

### Application areas

The medical device is used to support the external treatment of arthritis and to reduce the painful conditions of the hot and swollen joints.

### Dosing instructions, type and duration of application

When, how often and how long should you use Arthritis Gel?

Depending on the requirements, it can be used several times a day from the tube, applied and massaged to the relevant body area. Recommendation of dosage: apply 0,25 g size of a pea to hand, wrists or elbows, apply 0,5 g size of 2 peas to foot, ankle or knee. If no improvement is seen within 2-3 days after the treatment start or if the symptoms have worsened considerably, please consult a doctor.

The product is for temporary use.

People with an allergy to alcohol or an ingredient should not use the product.

### Please note

Keep out of reach of children.

There is no evidence against the gel use during pregnancy or lactation.

### Contraindications

Arthritis Gel should not be used in case of known hypersensitivity to any of the ingredients. Please do not use the product on the skin surface at the same time as others.

### Interactions

To avoid drug interactions, you should apply medications one to two hours apart or consult your doctor.

### Instructions and information on the durability of the medical device

Arthritis Gel may only be used up to the date marked " use by" on the carton.

### Pharmaceutical form and content

Original packaging with 50 ml gel.

### Composition

Active ingredient: alcohol / water mixture

### Other ingredients:

Menthol, Sodium Hydroxide Solution, cannabidiol, Acrylate Crosspolymer, Isopropyl Myristate, Camphor, Phytic acid

### Without preservatives.

Keep cool, dry and protected from sunlight. Small color changes have no influence on the efficacy of this product. Open tubes have to be used in 30 days.

### Example 12

### Instructions for use - psoriasis gel

### How does Psoriasis Gel work?

Psoriasis gel is a gel preparation with Dead Sea salt which
- relieves and supports external treatment of psoriasis
- promotes the desquamation process and a reduction of dandruff and dryness-related redness

Psoriasis Gel is a medical device. Efficacy and safety have been tested according to the European Directive on Medical Devices (93/42 / EEC).

### Principle of action of Psoriasis Gel

Psoriasis Gel is a topical formulation of Dead Sea salt in the form of a gel to be applied to the skin. The intended effect of the medical device is caused by the Dead Sea salt. The mode of action is to promote the desquamation process and to reduce dandruff and drought-related redness. After the gel is applied, an osmotic and a humectant effect (moisture retention effect) are produced. In this case, water is absorbed and retained in the gel by a hypertonic saline solution. This is supported by a physical conductive cooling effect from the ethanol. In addition, psoriasis gel contains main ingredients in the medical device, which improves skin hydration and thus counteracts new dandruff.

### Application areas

Psoriasis Gel is used to relieve and support external treatment of psoriasis with the typical symptoms such as dry, reddened and increased dandruff pron skin.

Dosing instructions, type and duration of application

When, how often and how long you should apply Psoriasis Gel?

Depending on requirements, a corresponding amount can be taken several times a day from the tube, applied and massaged to the relevant body area.

Recommendation of dosage: apply 0.25 g (size of a pea) to hand, wrists or elbows, apply 0.5 g (size of 2 peas) to foot, ankle or knee.

The gel must be washed off the surface of the skin within maximum one hour (to removed salt). Application and continuous application on the skin surface is possible up to 30 days.

If no improvement is seen within 2-3 days after the treatment start or if the symptoms have worsened considerably, please consult a doctor.

The product is for temporary use.

People with an allergy to an ingredient should not use the product.

### Please note

Keep out of reach of children.

There is no evidence against the gel use during pregnancy or lactation.

### Contraindications

Psoriasis Gel should not be used in case of known hypersensitivity to any of the ingredients. Please do not use the product on the skin surface at the same time as others.

### Interactions

To avoid drug interactions, you should apply medications one to two hours apart or consult your doctor.

### Instructions and information on the durability of the medical device

Psoriasis Gel may only be used up to the date marked " use by" on the carton.

### Pharmaceutical form and content

Original packaging with 50 ml gel.

### Composition

Active ingredient: Dead Sea Salt

### Other ingredients:

Ethanol/water, Panthenol, Tocopheryl Acetate, Hydroxyethylcellulose, Cannabidiol, Isopropyl Myristate, Retinyl Palmitate, Glycerin F, Aloe Barbadensis Leaf, Citric acid Gel contains alcohol.

This gel is contraindicated for use in persons with known allergy to any of the ingredients in it.

Keep cool and dry, protected from light.

## Claims

1. Hydroalcoholic gel composition formulated for topical use comprising:
a) cannabidiol present in an amount of 0.1-20%, such as 0.1-10% or more preferred 0.2-5 % (by weight);
b) a skin penetration enhancer present in an amount of 0.5-1.5% (by weight), such as a skin penetration enhancer is selected from isopropyl myristate, dimethylsulfoxid (DMSO), urea, or any combinations thereof;
c) ethanol present in an amount of 10-30 % (by weight);
d) one or more thickeners or gelling agents present in a total amount of 0.4-2 % (by weight), such as a thickener and/or gelling agent is selected from acrylate cross polymers, hydroxyethyl cellulose, xanthan gum and/or any combinations thereof; and
e) water in a quantity for the composition to a total of 100 % (by weight).

2. Composition according to claim 1, wherein cannabidiol used in preparation of the composition is obtained from natural Cannabis plants and is provided in a crystalline or pure form, and optionally, wherein the cannabidiol comprises less than 1.5 %, 1.0 %, or 0.5 % (by weight) of any one of: Cannabidivarin (CBDV), Cannabidiolic acid (CBDA), Cannabigerol (CBG), Cannabinol (CBN); and/or less than 1.0 % of Tetrahydrocannabinol (THC).

3. Composition according to claim 1 or 2, wherein the composition comprises no vegetable and/or mineral oil; and/or less than 5.0 %, 1.0 %, or 0.1 % (by weight) oil, such as vegetable and/or mineral oil.

4. Composition according to any one of the preceding claims, wherein the composition further comprises 10-25 % (by weight) of sodium chloride, in particular see salt, or more preferred Dead Sea salt.

5. Composition according to any one of the preceding claims, wherein the composition further comprises one or more skin care or skin hydrating/moisturizing agents, selected from:
i) 0.01-1 % (by weight) extract of aloe barbadensis leafs,
ii) 1 - 5 % (by weight) of panthenol,
iii) 0.1-1-5 % (by weight) of retinyl palmitate,
iv) 0.5-5 % (by weight) of glycerine,
or any combinations thereof.

6. Composition according to any one of the preceding claims, wherein the composition further comprises one or more further components selected from 0.5-5 % (by weight) of menthol and/or 0.1-2 % (by weight) of camphor and/or 0.5-1.5 % (by weight) of eucalyptus oil.

7. Composition according to any one of the preceding claims, comprising:
(I)
0.1-20, or 0.1-10 % (by weight) cannabidiol,
0.5-1.5 % (by weight) skin penetration enhancer or isopropyl myristate,
0.5-1.5 % (by weight) glycerine,
10-30 % (by weight) ethanol,
0.4-2 % (by weight) in total of one or more thickener(s) or gelling agent(s),
12-18 % (by weight) sodium chloride,
0.01-0.1 % (by weight) of extract of aloe barbadensis leafs,
1-5 % (by weight) panthenol,
0.1-1.5 % (by weight) retinyl palmitate, and
water in a quantity for the composition to a total of 100%;
(II)
0.5-2.0 % (by weight) cannabidiol,
0.3-1.5 % (by weight) skin penetration enhancer, in particular isopropyl myristate,
0.1-0.3 % (by weight) glycerine,
10-30 % (by weight) ethanol,
0.5-2.5 % (by weight) of one or more thickeners or gelling agents,
10-20 % (by weight) sea salt, preferably Dead Sea salt,
0.01-0.1 % (by weight) of extract of aloe barbadensis leafs,
1.5-4.0 % (by weight) panthenol,
0.1-0.5 % (by weight) retinyl palmitate,
0-01-0-03 % (by weight) antioxidant, and
water in a quantity for the composition to a total of 100% (by weight);
(III)
0.1-10, or 0.5-2.0 % (by weight) cannabidiol,
0.5-1.5% (by weight) skin penetration enhancer
0.5-1.5 % (by weight) glycerine,
10-20 % (by weight) ethanol,
0.4-2 % (by weight) one or more thickener(s) or gelling agent(s).
12-18 % (by weight) sodium chloride,
0.01-0.1 % (by weight) of extract of aloe barbadensis leafs"
2-3 % (by weight) panthenol,
0.1-0.5 % (by weight) retinyl palmitate, and
water in a quantity for the composition to a total of 100%;
(IV)
0.2 - 5, or 0.2-2.0 % (by weight) cannabidiol,
0.7-1.1 % (by weight) isopropyl myristate,
0.5-1.5 % (by weight) glycerine,
10-20 % (by weight) ethanol,
0.4-2 % (by weight) in total of one or more thickener(s) or gelling agent(s),
12-18 % (by weight) Dead Sea salt,
0.01-0.1 % (by weight) of extract of aloe barbadensis leafs,
2-3 % (by weight) of panthenol (of 75% purity),
1.0-3.0 % (by weight) tocopherol acetate
0.1-0.5 % (by weight) retinyl palmitate,
0.01-0.03 % (by weight) sodium stearoyl glutamate,
0.01-0.03 % (by weight) of citric acid (monohydrate), and
water in a quantity for the composition to a total of 100%;
(V)
0.1-10.0 % (by weight) cannabidiol,
0.5-1.5% (by weight) skin penetration enhancer(s),
20-30 % (by weight) ethanol,
0.5-1.5 % (by weight) in total of one or more thickener(s) or gelling agent(s),
1.5-2.5 % (by weight) menthol,
0.4-0.7 % (by weight) of camphor, and
water in a quantity for the composition to a total of 100%;
or
(VI)
0.2-5 0, or 0.2-2 % (by weight) cannabidiol,
0.7-1.1 % (by weight) skin penetration enhancer(s)
20-30 % (by weight) ethanol,
0.5-1.5, or 0.8-1.2 % (by weight) in total of one or more thickener(s) or gelling agent(s),
1.5-2.5 % (by weight) menthol,
0.4-0.7 % (by weight) of camphor,
0.05-0.3 % (by weight) phytic acid (of 50% purity), and
water in a quantity for the composition to a total of 100 %.

8. Composition according to any one of the preceding claims, wherein the composition further comprises one or more pharmaceutically acceptable adjuvants selected from antioxidants, emulsifiers, pH regulating agents, such as acids or bases, stabilizers, colorants, or any combination thereof.

9. Composition according to any one of the preceding claims, wherein the composition is a cosmetic skin hydrating or skin care composition.

10. Composition according to any one of the preceding claims for use as a medicament.

11. Composition for use according to claim 10 for use as:
- a medical composition in a local topical application in the treatment or alleviation of symptoms, in particular pain, resulting from arthritis, in particular rheumatoid arthritis, osteoarthritis, juvenile rheumatoid arthritis and/or psoriatric arthritis and/or neurological pain, such as pain resulting from sclerosis, e.g. multiple sclerosis, in a subject, such as a human, mammal or other animal; and/or
- medical composition to alleviate a painful condition associated with one or more hot and/or swollen joints in a subject, such as a human, mammal, or other animal.

12. Composition for use according to claim 10 or 11, for use as a medical composition in local a topical application in the treatment or alleviation of symptoms of psoriasis, such as mild, moderate, and/or severe psoriasis, in particular in the treatment or alleviation of red, dry, itchy, and/or scaly skin, in a subject, such as a human, mammal, or other animal.

13. Composition for use according to any one of claim 10-12, wherein the topical gel composition is applied topically to a local skin area of a subject, such as a human, mammal or other animal, 1-5 times daily; and/or wherein the topical gel composition is applied topically to a local skin area of a subject in an amount of 5-100, or 10-50 mg/ cm² per application.

14. A kit comprising a composition according to any one of claims 1-9, or a composition for use according to any one of claims 10-13, in a container, and optionally an instruction for use.

15. A method for providing a hydroalcoholic gel composition according to any one of claims 1-9, or a composition for use according to any one of claims 10-13, said method comprising:
a) Dissolving the cannabidiol in ethanol to provide a cannabidiol-containing ethanol fraction;
b) Providing an aqueous fraction comprising the remaining ingredients; and
c) Mixing the cannabidiol-containg ethanol fraction from step (a) with the aqueous fraction from step (b) to provide the hydroalcoholic gel.
